# EUROPEAN PATENT APPLICATION

(11) **EP 1 258 527 A1**
(43) Date of publication of application: **20.11.2002**
(21) Application number: 01201616.8
(22) Date of filing: 02.05.2001
(51) Int. Cl.: C12N 9/16, C07K 1/16, C07K 16/40

(54) **Membrane-associated shingomyelinase derived from bovine brain, isolation method therfor and anti-sphingomyelinase monocional antibody**

(71) Applicant: SK Corporation, Seoul 110-110 (KR); Kim, Dae-Kyong, Seocho-ku, Seoul 137-069 (KR)
(72) Inventor: Kim, Dae-Kyong, Seocho-ku, Seoul 137-069 (KR); Jung, Sung-Yun, Munheung-dong, Buk-ku, Kwangju-si500-110 (KR); Jung, Kwang-Mook, Bundang-ku, Sungnam-si (KR)
(74) Representative: Long, Giorgio

(57) **Abstract**

Disclosed is a sphingomyelinase derived from the neuron membrane of the bovine brain, which is 60 kDa in molecular weight and Mg²⁺-dependent in activity with an optimal pH ranging from 6.0 to 9.0. It is prepared by homogenizing a bovine brain tissue and centrifuging the homogenate to remove cell debris and nuclei; separating cell membranes from the supernatant into a pellet through centrifugation; treating the cell membrane pellet with a buffer containing ammonium sulfate to disassociate the sphingomyelinase from the membrane; subjecting the extract to anion exchange chromatography; sonicating the chromatography fraction in a buffer containing Triton X-100 and centrifuging the sonicated fraction to obtain a supernatant containing the sphingomyelinase; and purifying the sphingomyelinase by subjecting the supernatant to hydrophobic interaction chromatography, anion exchange high-performance liquid chromatography, hydrophobic interaction high performance liquid chromatography, and cation exchange fast protein liquid chromatography, in due order. Also, disclosed is a monoclonal antibody against the sphingomyelinase, produced by Hybridoma SM1A5 (Deposition No. KCLRF-BT-00025).

## Description

### BACKGROUND OF THE INVENTION

### 1. Field of the invention

The present invention relates to a membrane-associated, neutral pH optimum sphingomyelinase, a method of isolating the same, and a monoclonal antibody against the same.

### 2. Description of the Prior Art

Serving as a secondary transmitter in signal transduction, ceramide is involved in a variety of cellular responses, including cell differentiation, cell cycle suspension, cell aging, apoptosis, etc. Ceramide is produced chiefly as a result of the hydrolysis of sphingomyelin, a membrane phospholipid.

Sphingomyelinase (SMase) functions to hydrolyze sphingomyelin into ceramide and phosphocholine and various isozymes thereof have been found: liposomal, acidic pH optimum SMase (A-SMase); cytoplasmic, Zn-dependent, acidic pH optimum SMase; alkaline pH optimum SMase; cytoplasmic, Mg²⁺-dependent, neutral pH optimum SMase (N-cSMase); and membrane-associated, Mg²⁺-dependent, neutral pH optimum SMase (N-mSMase). A-SMase and neutral type SMases are known to increase the concentration of cytoplasmic ceramide in response to extracellular stimuli, such as 1α, 25-dihydroxyvitamin D₃, tumor necrosis factor-α, interleukin-1β, nerve growth factors, chemotherapy drugs, blood plasma depletion, etc.

Recently, acidic pH optimum SMase derived from human urine has been isolated and cDNA coding for this enzyme was successfully cloned (Quintern, L. E., et al., EMBO J., 8, 2469-2473 (1989)). The present inventors also isolated and characterized a cytoplasmic 75 kDa N-cSMase whose activity is dependent on Mg²⁺ and sensitive to glutathione and DTT, whose contents were applied for a patent (Korean Pat. Appl'n No. 98-28187).

Meanwhile, most N-mSMases have not yet been fully characterized because they are very difficult to isolate owing to their high hydrophobicity. Despite such difficulties, an N-mSMase with a molecular weight of 47.5 kDa was cloned from mammalian cells (Tomiuk, S., et al., Proc. Natl. Acad. Sci. USA, 95, 3638-3643 (1998)). However, this N-mSMase, although being a membrane-associated protein which can be dissociated by use of Triton X-100, is described as not being involved in the TNF-α signal transduction pathway. Another N-mSMase was successfully isolated from rat brain by use of Triton X-100, but in a partially purified form (Liu, B., et al., J. Biol. Chem., 273, 34472-34479 (1988)). It is reported that the activity of the partially purified N-mSMase is inhibited by glutathione (concentration at which enzyme activity is 50 % inhibited (IC₅₀): 2.5 mM) and promoted by DTT and phosphatidylserine. The present inventors succeeded in fully isolating from bovine brain two groups of novel N-mSMases; one group with a molecular weight of 35 kDa consisting of four isoenzymes (T-mSMases A, B, C and D) by use of Triton X-100; and the other group with a molecular weight of 40 kDa consisting of two isoenzymes (S-mSMases I and II) by use of ammonium sulfate and characterized all of them, which are under patent-pending (Korean Pat. Appl'n No. 98-28187).

Particularly, the N-mSMases which exist in brain tissues are believed to play an important role in the brain aetiology, since the signal transduction pathways mediated by ceramide in brain tissues are responsible for nerve cell death resulting in Parkinson's disease or Alzheimer's disease, the apoptosis of oligodendrocytes, PC12 cells and T9 cells, all involved in the mediation of low affinitive neurotrophin receptor (p75NTR), the oligodendrocyte apoptosis caused by TNF-α, and the pathology of some central nervous system diseases such as ischemic brain damages.

### SUMMARY OF THE INVENTION

Therefore, it is an object of the present invention to provide a membrane-associated, neutral pH optimum sphingomyelinase isolated from bovine brain, which is useful for the development of curing agents for brain diseases, such as Parkinson's disease, Alzheimer's diseases, etc.

It is another object of the present invention to provide a method for preparing such a sphingomyelinase from bovine brain.

It is a further object of the present invention to provide a monoclonal antibody specific for the sphingomyelinase.

In accordance with a first embodiment of the present invention, there is provided a sphingomyelinase derived from the neuron membrane of the bovine brain, which is 60 kDa in molecular weight and Mg²⁺-dependent in activity with an optimal pH ranging from 6.0 to 9.0.

In accordance with a second embodiment of the present invention, there is provided a method for isolating the sphingomyelinase according to the present invention, comprising the steps of: homogenizing a bovine brain tissue and centrifuging the homogenate to remove cell debris and nuclei; separating cell membranes from the supernatant into a pellet through centrifugation; treating the cell membrane pellet with a buffer containing ammonium sulfate to disassociate the sphingomyelinase from the membrane; subjecting the extract to anion exchange chromatography; sonicating the chromatography fraction in a buffer containing Triton X-100 and centrifuging the sonicated fraction to obtain a supernatant containing the sphingomyelinase; and purifying the sphingomyelinase by subjecting the supernatant to hydrophobic interaction chromatography, anion exchange high-performance liquid chromatography, hydrophobic interaction high performance liquid chromatography, and cation exchange fast protein liquid chromatography, in due order.

In accordance with a third embodiment of the present invention, there is provided a monoclonal antibody against the sphingomyelinase, which is produced by Hybridoma SMlA5 (Deposition No. KCLRF-BT-00025).

### BRIEF DESCRIPTION OF THE DRAWINGS

Fig. 1 is a chromatogram drawn after the DEAE-cellulose anion exchange chromatography of an ammonium sulfate extract of bovine brain;
Fig. 2 is a chromatogram drawn after the butyl-Toyopearl 650M hydrophobic interaction chromatography of the active fractions obtained through the anion exchange chromatography;
Fig. 3 is a chromatogram drawn after the DEAE-5PW anion exchange HPLC of the active fractions obtained through the hydrophobic interaction chromatography;
Fig. 4 is a chromatogram drawn after the phenyl-5PW hydrophobic interaction chromatography of the active fractions obtained through the anion exchange HPLC;
Fig. 5 is a chromatogram drawn after the mono S cation exchange FPLC of the active fractions obtained through the hydrophobic interaction HPLC;
Fig. 6 shows SDS-PAGE results of Peak α (a) and Peak β (b) obtained through the cation exchange FPLC;
Fig. 7 shows an immunoprecipitation result for the sphingomyelinase of the present invention;
Fig. 8 shows a Western blotting result for the sphingomyelinase of the present invention;
Fig. 9 shows Lineweaver-Burk plots in which reciprocals of the velocity of the sphingomyelinase is plotted against the reciprocals of substrate concentrations in absence of surfactants (a) and in the presence of 0.1, 1.0 and 2.0 mM sodium deoxycholate (b);
Fig. 10 is a graph in which the activity of the sphingomyelinase of the present invention is plotted against Mg²⁺ concentrations;
Fig. 11 is a graph in which the activity of the sphingomyelinase of the present invention is plotted against pH changes;
Fig. 12 is a graph in which the activity of the sphingomyelinase of the present invention is plotted against the concentrations of Ca²⁺, Fe²⁺, Fe³⁺, Zn²⁺ and Cu²⁺;
Fig. 13 shows graphs in which the activity of the sphingomyelinase of the present invention is plotted against the concentrations of GSH, DTT and 2-mercaptoethanol (a) and against the concentration of GSSG, cystein, cystein-glycine and γ-glutaric acid-cystein;
Fig. 14 shows graphs in which the activity of the sphingomyelinase of the present invention is plotted against the concentrations of sodium deoxycholate (a) and Triton X-100 (b); and
Fig. 15 shows an SDS-PAGE result of the neuron membrane fraction obtained from bovine brain.

### DETAILED DESCRIPTION OF THE INVENTION

The present invention pertains to a membrane-bound protein isolated from the nerve cell membrane of the bovine brain, which has the enzymatic activity of hydrolyzing sphingomyelin into ceramide and phosphocholine.

Versus sphingomyelin, the sphingomyelinase of the present invention shows high enzymatic activity with Km of 47 µM and Vmax of 1,587 nmol/min/mg, which is 50 times higher than the activity when other phospholipids are used as substrates.

In addition to being dependent on Mg²⁺, the sphingomyelinase is active in the pH range of 6.0 to 9.0 with optimum activity at pH 7.5. In addition, the activity of the sphingomyelinase of the present invention is affected by cation. While Cu²⁺, Zn²⁺, and Fe²⁺ all inhibit enzymatic activity (hydrolysis of sphingomyelin) (IC₅₀ values 28 µM, 32 µM and 47 µM, respectively), enzymatic activity is increased by a factor of 1.8 in the presence of Fe³⁺. Interestingly, the activity of the sphingomyelinase is reduced by Fe²⁺, but improved by Fe³⁺.

Another group of compounds capable of affecting the activity of the sphingomyelinase of the present invention includes glutathione, oxidized glutathione, cystein, cystein-glycine, and γ-glutaric acid-cystein. With respect to glutathione concentration, the sphingomyelinase of the present invention exhibits two different behaviors. The activity of the sphingomyelinase increases as the concentration of glutathione increases up to 3 mM. On the other hand, the activity of the sphingomyelinase decreases with an increase in glutathione concentration above 3 mM and is completely inhibited at a glutathione concentration of 10 mM (IC₅₀ 8.0 mM). As for GSSG, cystein, cystein-glycine, and γ-glutaric acid-cystein, an increase in the concentration of each of them brings about a reduction in the activity of the sphingomyelinase. Particularly, the sensitivity of the sphingomyelinase of the present invention to glutathione depletion is far greater than that of the partially purified N-mSMase (IC₅₀ 2.5 mM) of Liu's (Liu, B., et al., J. Biol. Chem., 273, 34472-34479 (1998)). In contrast, neither dithiothreitol nor 2-mercaptoethanol has any influence on the activity of the sphingomyelinase.

Such surfactants as sodium deoxycholate and Triton X-100 belong to another group which can affect the activity of the sphingomyelinase of the present invention. At 1 mM sodium deoxycholate, the sphingomyelinase of the present invention exhibits an optimal activity which is 30 times higher than that in the absence of sodium deoxycholate. The presence of 0.005 % Triton X-100 makes the sphingomyelinase of the present invention have optimal activity half as high again as that when no Triton X-100 is present.

The sphingomyelinase of the present invention is found in the membranes of neurons in the cortex of bovine brain, but not in neuroglial cells. The sphingomyelinase is believed to be a kind of a peripheral protein bound to the membrane via an ionic bond, inferred from the fact that it is of very high hydrophobicity and can be solubilized from membranes by sodium chloride or ammonium sulfate.

By taking advantage of these characteristics, the sphingomyelinase of the present invention can be purified as follows.

To begin with, a bovine brain tissue is homogenized in an ordinary manner, followed by the centrifugation of the homogenate to remove cell debris and nuclei. The supernatant is centrifuged again to obtain a cell membrane pellet. On this cell membrane pellet, an ordinary protein isolation method is conducted using a buffer containing ammonium sulfate. In this time, sphingomyelinases, if isolated by Triton X-100, are not isolated by ammonium sulfate. The ammonium sulfate extract thus obtained is used as a sphingomyelinase source in subsequent steps.

Next, the ammonium sulfate extract is subjected to anion exchange chromatography. Examples of the columns useful in the anion exchange chromatography include DEAE-cellulose (Whatman Biosystem Ltd., England), QAE-cellulose (Whatman Biosystem Ltd., England), DEAE-cephalose (Pharmacia Biotechnology Inc., Sweden), QAE-cephalose (Pharmacia Biotechnology Inc., Sweden), DEAE-Toyopearl (Tosoh Co., Japan), and QAE-Toyopearl (Tosoh Co., Japan) with preference to DEAE-cellulose. To be useful in chromatography, a DEAE-cellulose column is pre-equilibrated with buffer D (25 mM Tris-HCl, pH 7.5, 1 mM EDTA and 10 mM 2-mercaptoethanol). Following the loading of the ammonium sulfate extract on the equilibrated DEAE-cellulose column, elution with a buffer D containing 0.5 M ammonium sulfate and 0.1 % Triton X-100 is conducted. The active fraction is read in a single peak.

After being sonicated in a buffer containing Triton X-100 to solubilize sphingomyelinase, the active fraction is deprived of insoluble lipid components by centrifugation. This step is very important for the effective recovery of sphingomyelinase. If this step is omitted then the membrane-associated sphingomyelinase of high hydrophobicity aggregates with other lipid components and it is quite difficult to recover the sphingomyelinase from the aggregate. After being added with an ammonium sulfate solution, the supernatant free of insoluble lipid components is subjected to hydrophobic interaction chromatography. Columns useful in this chromatography can be exemplified by Butyl-Toyopearl 650 M (Tosoh co., Japan), Phenyl-Toyopearl 650M (Tosoh Co., Japan), Butyl-cephalose (Pharmacia Biotechnology Inc.), and Phenyl-cephalose (Pharmacia Biotechnology Inc). Of them, the Butyl-Toyopearl 650 M column is preferred. To be useful in the hydrophobic interaction chromatography, the Butyl-Toyopearl 650 M column is pre-equilibrated with a buffer D containing 0.5 M ammonium sulfate. On the pre-equilibrated column is loaded the supernatant containing ammonium sulfate, after which the sphingomyelinase can be eluted with a buffer D containing 0.2 M ammonium sulfate and then with water. The active fraction is read in a single peak.

Afterwards, the active fraction is further purified through anion exchange high performance liquid chromatography (HPLC). For anion exchange HPLC, commercially available columns, such as DEAE-5PW (Tosho Co., Japan), DEAE-5PW (Tosoh Co., Japan), Mono Q (Pharmacia Biotechnology Inc., Sweden), Mono P (Pharmacia Biotechnology Inc., Sweden), Resource Q (Pharmacia Biotechnology Inc., Sweden) may be used with preference for DEAE-5PW. Prior to being used in the chromatography, the DEAE-5PW HPLC is equilibrated with a buffer D. On the equilibrated column is loaded the active fraction, followed by the elution with a linear gradient of a buffer D containing 0.5 M ammonium sulfate and 0.1 % Triton X-100. An active fraction is read in a single peak.

Next, hydrophobic interaction HPLC is applied to the active fraction thus obtained. Columns suitable for use in this hydrophobic interaction HPLC may be exemplified by Phenyl-5PW, Phenyl-5PW glass, Ether-5PW glass, Ether-5PW, Butyl-NPR (Tosoh Co., Japan), SOURCE 15ETH, SOURCE 15ISO, SOURCE 15PHE, Phenyl Cephalose High Performance, Butyl Cephalose 4 Fast Flow, Octyl Cephalose 4 Fast Flow, and Phenyl Cephalose 6 Fast Flow (Pharmacia Biotechnology Inc), of which Phenyl-5PW column is preferred. Before the loading of the active fraction, a Phenyl-5PW column is equilibrated with buffer D containing 0.2 M ammonium sulfate. Elution utilizes a linear gradient of distilled water. Active fractions of interest are read in two peaks, designated peaks ε and ζ, respectively.

Finally, the active fractions thus obtained are subjected to cation exchange fast protein liquid chromatography (FPLC). Examples of the columns suitable for use in this FPLC include Mono S FPLC (Pharmacia Biotechnology Co., Sweden), Resource S (Pharmacia Biotechnology Co., Sweden), and SP-5PW (Tosoh Co., Japan) with preference for the Mono S FPLC column. To be active, the Mono S cation exchange FPLC column is pre-equilibrated with a buffer S (25 mM sodium acetate pH 6.5 and 1 mM EDTA). On the equilibrated FPLC column is loaded a mixture of the active fraction and the buffer S, followed by elution with a linear gradient of sodium chloride and buffer S containing 1.0 M sodium chloride and 0.1 % Triton X-100, in that order. Active fractions of interest are read in two peaks, designated peaks A and B, respectively.

Taken together, the data obtained by SDS-PAGE, immunoprecipitation, and Western blotting analyses demonstrate that each of the active fractions appearing in peaks A and B contain the present sphingomyelinase with a molecular weight of 60 kDa. Also, the active fraction of the peak B was found to be 1.4 fold greater in total activity and 4 fold greater in specific activity than that of the peak A. The sphingomyelinase thus obtained according to the present invention is named N-mSMase ε.

Also, the present invention pertains to a monoclonal antibody against the sphingomyelinase. In order to obtain the monoclonal anti-sphingomyelinase antibody, a hybridoma cell, named SM1A5, was prepared following a known technique. The hybridoma SM1A5 capable of producing the monoclonal antibody was deposited in the Korean Cell Line Research Foundation (KCLRF) on September 15, 1999, with a deposition No. KCLRF-BP-00025.

A better understanding of the present invention may be obtained in light of the following examples which are set forth to illustrate, but are not to be construed to limit the present invention.

### EXPERIMENTAL EXAMPLE 1

### N-mSMase Activity Assay

After being dried with a nitrogen stream, [N-methyl-¹⁴ C]sphingomyelin (choline residue labeled with ¹⁴C) was suspended in ethanol. 10 µl of a sample was mixed with 100 µl of a reaction solution (10 mM MgSO₄, 50 µM [N-methyl-¹⁴ C]sphingomyelin (c.a. 60,000 cpm), 2 mM sodium deoxycholate, 100 mM Tris-HCl (pH 7.0)) and incubated at 37 °C for 30 min. Addition of 320 µl of a mixture of chloroform/methanol (1:1 volume ratio) and 30 µl of 2N hydrochloride stopped the reaction. After vortexing and centrifuging the resulting mixture at 10,000xg, 200 µl of the aqueous layer was mixed with 2.5 ml of a scintillation cocktail, such as that manufactured by Packard Instrument Co., U.S.A., identified as "Insta gel-XF", followed by measuring the radioactivity with the aid of a Packard Tricarb liquid β-scintillation counter.

### EXPERIMENTAL EXAMPLE 2

### Quantitative Protein Assay

Proteins were quantified by measuring the absorbance at 280 nm in a UV detector (Pharmacia Biotechnology Co., Sweden). In this regard, after the Bradford reagent was used to dye the protein contained in a sample, its protein concentration was determined by comparing the measured absorbance to a standard curve drawn on the basis of a bovine serum albumin concentration gradient.

### EXAMPLE 1

### Purification of N-mSMase ε

### Step 1: Preparation of Enzyme Source

5 kg of a bovine brain was homogenized in 25 liters of a homogenization buffer (50 mM Tris-HCl pH 7.5, 1 mM EDTA, 3 mM MgCl₂, 50 mM KCl and 10 mM 2-mercaptoethanol) using a homogenizer, such as that manufactured by Kinematica, Switzerland, identified as Polytrom Homogenizer Model PT-MR 6000). The homogenate was centrifuged at 10,000xg for 10 min and the supernatant free of cell debris and nuclei was centrifuged again at 10,000xg at 4 °C for 1 hour. The pellet was suspended in 2.5 liters of the homogenization buffer, followed by centrifugation at 40,000xg at 4 °C for 1 hour. The pellet thus obtained was re-suspended in 2.5 liters of a homogenization buffer containing 0.5 M ammonium sulfate and the suspension was stirred at 4 °C for 1 hour, followed by centrifugation at 4 °C at 40,000xg for 1 hour. The supernatant (ammonium sulfate extract) was harvested and used as an enzyme source from which N-mSMase would be purified.

### Step 2: DEAE-Cellulose Anion Exchange Chromatography

A DEAE-cellulose column filled with 2.0 L of DE52 gel (Whatman Biosystem Ltd., England) was pre-equilibrated with a buffer D (25 mM Tris-HCl pH 7.5, 1 mM EDTA and 10 mM 2-mercaptoethanol) and 2.5 L of the ammonium sulfate extract prepared in Step 1 was loaded on the equilibrated column. By adding a buffer D containing 0.5 M ammonium sulfate and 0.1 % Triton X-100 stepwise, the supernatant loaded was eluted at a flow rate of 20 ml/min while taking a fraction every 40 ml. 10 µl of each fraction was taken and analyzed for N-mSMase activity in the manner described in Experimental Example 1.

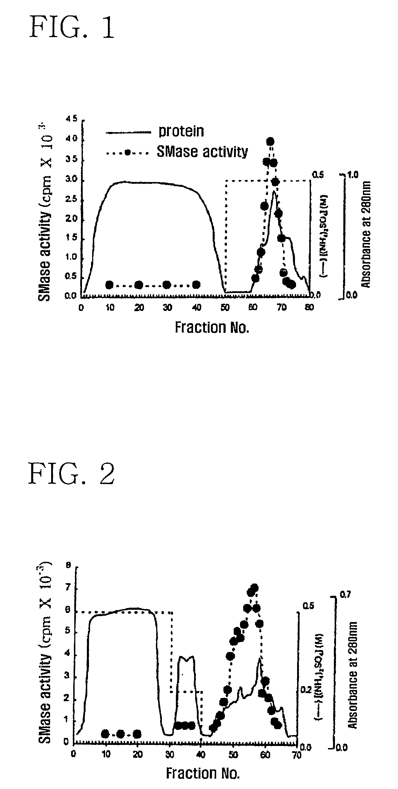

Results of the chromatography are shown in Fig. 1. As seen in the chromatogram, the N-mSMase activity was read in a single peak.

### Step 3: Butyl-Toyopearl 650 M Hydrophobic Interaction Chromatography

The active fraction obtained in Step 2 was sonicated six times at 4 °C at intervals of 5 sec in a cell crusher (Sonics & Materials Inc., Canbury, U.S.A.) at a power at 70 % of the amplitude for 3 sec per sonication round, followed by centrifugation at 100,000xg for 1 hour. With the supernatant thus obtained, a 4.0 M ammonium sulfate solution was diluted to a 0.5 M sample.

This sample was loaded on a Butyl-Toyopearl 650 M column (Tosoh Co., Japan, bed volume 150 ml), followed by adding buffer D containing 0.2 M ammonium sulfate stepwise and then distilled water. Elution was conducted at a flow rate of 15 ml/min while collecting a fraction every 30 ml. 10 µl of each fraction was taken and analyzed for N-mSMase activity in the manner described in Experimental Example 1, so as to determine active fractions.

Elucidating the analysis results, a chromatogram is given in Fig. 2. As seen in the chromatogram, the N-mSMase activity was read in a single peak.

### Step 4: DEAE-5PW Anion Exchange HPLC

On a DEAE-5PW HPLC column (21.5mmx15cm, Tosoh Co., Japan) which had been equilibrated with buffer D, the active fraction obtained in Step 3 was loaded, followed by eluting with a linear gradient of 200 ml of buffer D containing 0.5 M ammonium sulfate and 0.1% Triton X-100 at a flow rate of 5 ml/min while collecting a fraction every 5 ml. 3 µl of each fraction was taken and assayed for N-mSMase activity in the manner described in Experimental Example 1 to obtain an active fraction.

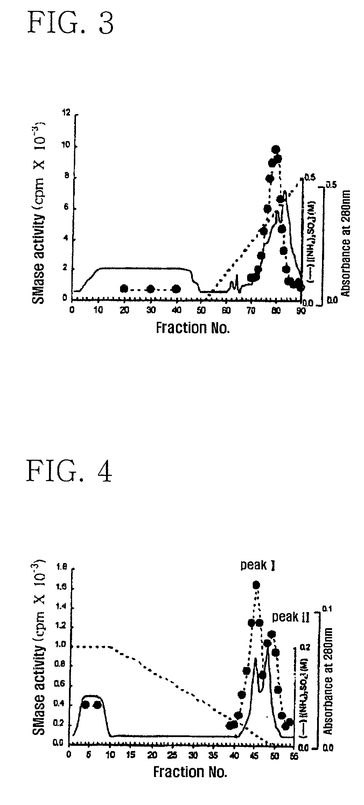

The results are shown in Fig. 3. As seen in the chromatogram, the N-mSMase activity was read in a single peak.

### Step 5: Phenyl-5PW Hydrophobic Interaction HPLC

On a phenyl-5PW HPLC column (21.5mmx15cm, Tosoh Co., Japan) which had been equilibrated with buffer D, the active fraction obtained in Step 4 was loaded, followed by eluting with a linear gradient of distilled water at a flow rate of 5 ml/min while collecting a fraction every 5 ml. 3 µl of each fraction was taken and assayed for N-mSMase activity in the manner described in Experimental Example 1 to obtain active fraction.

The results are shown in Fig. 4. As seen in the chromatogram, the N-mSMase activity was read in two peaks, which were named peaks ε and ζ, respectively.

### Step 6: Mono S Cation Exchange FPLC

Only the fractions of the peak ε were collected, mixed with the same volume of buffer S (25 mM sodium acetate pH 6.5 and 1 mM EDTA), and loaded on a Mono S cation exchange FPLC column (5.0 cm x 5.0 mm, Pharmacia Biotechnology Co., Sweden) which was pre-equilibrated with buffer S, followed by eluting with 20 ml of sodium chloride with a linear gradient from 0.0 to 1.0 M and then with a stepwise gradient of buffer S containing 1.0 M sodium chloride and 0.1 % Triton X-100 at a flow rate of 1 ml/min while collecting a fraction every 1 ml. 3 µl of each fraction was taken and analyzed for N-mSMase activity in the manner described in Experimental Example 1, so as to determine which fractions contained enzymatic activity.

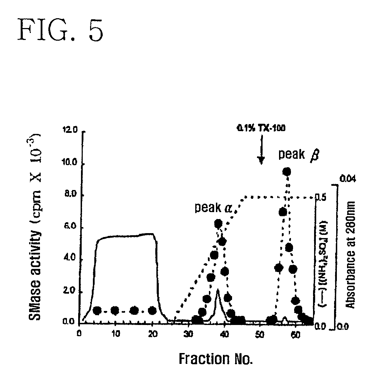

Elucidating the analysis results, a chromatogram is given in Fig. 5. As seen in the chromatogram, the N-mSMase activity was read in two peaks, which were named peaks A and B. The N-mSMase obtained from these active peaks was named N-mSMase ε.

Summarized in Table 1, below, are total quantities, total activities, yields, specific activities and purities of the proteins obtained in each step.

**TABLE 1**

| Purification Step | Total Protein (mg) | Total Activity (nmol/min) | Yield (%) | Specific Activity (nmol/min/mg) | Purity (factor) |
|---|---|---|---|---|---|
| 40,000xg pellet | 6250 | 895 | 100 | 0.15 | 1 |
| Ammonium Sulfate Extract | 2500 | 1440 | 161 | 0.60 | 4 |
| DEAE-Cellulose | 532 | 630 | 70.4 | 1.20 | 8 |
| Sonication Extract | 352 | 545 | 60.9 | 1.55 | 10 |
| Butyl-Toyopearl | 12 | 240 | 26.8 | 20.00 | 133 |
| DEAE-5PW HPLC | 3.70 | 95 | 10.6 | 25.70 | 171 |
| Phenyl-5PW HPLC Peak ε | 0.27 | 32 | 3.6 | 118.50 | 790 |
| Phenyl-5PW HPLC Peak ζ | 0.38 | 21 | 2.3 | 55.25 | 368 |
| Mono S FPLC Peak A | 0.03 | 9 | 1.0 | 300.00 | 2000 |
| Mono S FPLC Peak B | <0.01 | 11.5 | 1.3 | >1150.00 | >7667 |

### EXAMPLE 2

### SDS-PAGE

Proteins were analyzed by SDS-PAGE in accordance with the Laemmli method (U.K., Nature, 227, 680-685 (1970)). 10 µl of each of the active fractions with Peaks A and B, respectively, obtained in Step 6 of Example 1, were mixed with 2 µl of 5x Laemmli buffer (0.125 M Tris-HCl pH 6.8, 4 % SDS, 20 % glycerol and 0.002 % bromophenol blue), boiled for 5 min and cooled to room temperature, followed by running the samples on a 10% SDS-polyacrylamide gel. For visualization, separated proteins were dyed using the PlusOne Silver Staining Kit (Pharmacia LKB Co., Sweden).

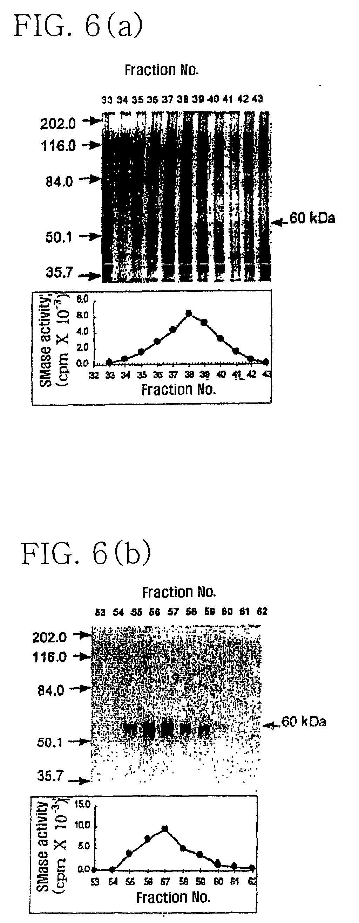

The electrophoresis results are given in Figs. 6a (Peak A) and 6b (Peak B), each of which employed a standard protein marker composed of myosin (22 kDa), β-galactosidase (116 kDa), bovine serum albumin (84 kDa), ovalbumin (50.1 kDa), carbonic anhydrase (35.7 kDa) as indicated by arrows on the left side. On lanes 1 to 11 of Fig. 6a were electrophoresed the active fractions of Peak A shown in Fig. 5 (Fraction Nos. 33 to 43), respectively. In Fig. 6b, lanes 1 to 10 showed proteins separated from active fractions of Peak B obtained in Fig. 5 (Fraction Nos. 53 to 62), respectively. As shown in Figs. 6a and 6b, all the N-mSMase ε separated from the fractions of Peaks A and B were found to be approximately 60 kDa and to be the only proteins therein.

### EXAMPLE 3

### Production of Monoclonal Antibody Against N-mSMase ε

50 µg of the N-mSMase ε obtained in Step 6 of Example 1 was peritoneally injected, along with a Freund's complete adjuvant (Gibco BRL Life Technologies, Inc., U.S.A.), to BALB/c mice (purchased from Korean Experimental Animal Center, Korea). Additional immunization was carried out by injecting N-mSMase ε, which was emulsified in Freund's incomplete adjuvant, at a dose of 50 µg three more times at intervals of three weeks. Three days before fusion, N-mSMase ε was finally injected at a dose of 50 µg without adjuvants, followed by sampling blood from the mice. Centrifugation at 10,000xg for 30 min gave anti-sera.

Spleen cells from the mice (2x10⁸ cells) were fused with myeloma cells from SP2/O mice using 43% polyethyleneglycol (PEG) 50 (Sigma Co., U.S.A.) according to the Goding's protocol (Goding, J. W., in Monoclonal Antibodies; Principles and Practice, Academic Press, London (1986)), followed by plating them on 96-well plates containing a HAT medium (Sigma Co., U.S.A.) supplemented with 10% hybrid promoting medium (Sigmal Co., U.S.A.), 20% fetal bovine serum (Hyclone), 35 % SP20 and 35 % HEFM buffer and RPMI-1640 (Gibco BRL, Grand Island, NY., U.S.A.). After culturing at 37 °C for 2 weeks, the hybridoma supernatant was used to determine whether the cells produced anti-N-mSMase ε antibodies in accordance with Ausubel's instructions (Ausubel, F. M., in Current Protocols in Molecular Biology, Willey-Interscience, New York (1987)). This provided three hybridoma clones, named SM1A5, SM1H6 and SM1E1, which could produce monoclonal antibodies against N-mSMase ε. SM1A5 was deposited in the Korean Cell Line Research Foundation (KCLRF) on September 15, 1999, with a deposition No. KCLRF-BT-00025.

### EXAMPLE 4

### Immunoprecipitation of N-mSMase ε

25 µl of the anti-serum obtained in Example 3, 500 µl of the medium for hybridoma SM1A5, SM1H6 or SM1E1, and 25 µl of serum obtained before the immunization were each reacted at 4 °C for 12 hours with 50 µl of protein A-cephalose CL-4B beads (Pharmacia Biotechnology Inc., Sweden) while agitating. In advance of this reaction, the beads were equilibrated with buffer I (20 mM Tris-HCl pH 7.5, 1 mM EDTA and 10 % BSA (20:1 v/v)). After being washed six times with 1 ml of buffer I, the beads were reacted with a solution of the N-mSMase ε (100 ng) obtained in Step 6 of Example I, in buffer I (100 µg) at 4 °C for 0, 15, 30, 45 or 60 min with agitation. Following centrifugation at 4 °C at 13,000xg for 30 sec, the supernatant was analyzed for N-mSMase activity according to the method of Experimental Example 1.

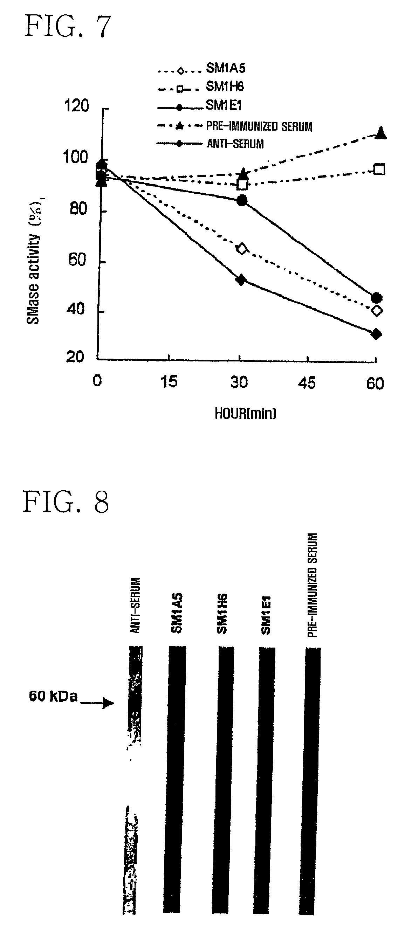

The results are depicted in Fig. 7. As seen, antibodies produced from hybridoma clones SM1A5 and SM1E1 decreased the activity of N-mSMase ε to similar degrees, indicating that they had similar affinity for the antigen.

### EXAMPLE 5

### Western Blotting

About 100 ng of the active fraction obtained in Step 5 of Example 1 was electrophoresed on 10% SDS-polyacrylamide gel and the separated protein was electrically transferred onto a nitrocellulose membrane at 200 mA by means of a Hoefer electroblotter system (TE22 Mighty Small Transphor Unit, Hoefer Scientific Instruments, Canada). This cellulose membrane was treated with a solution of 5% non-fat dry milk in Tris-buffered saline (25 mM Tris-HCl pH 8.0, 137 mM NaCl and 2.7 mM KCl) to block the transferred proteins, washed with TBS containing 0.1 % Tween 20, and incubated for 6 hours, together with the antiserum, a medium for hybridoma SM1A5, SM1H6 or SM1E1, or a pre-immunized serum used as a control. After being washed with TBS containing 0.1 % Tween-20, the proteins transferred onto the membrane were reacted with a goat anti-mouse IgG-alkaline phosphatase conjugate (Santa Cruz Biotechnology, U.S.A.) (dilution factor 1:2,000) for 2 hours, washed in the manner described above, and viewed through a coloring reaction with a substrate kit (MBT/BCIP, Pierce, U.S.A.). For the hybridoma media, TBS alone was used as a washing buffer.

Blotting Results are given in Fig. 8. As can be seen, the monoclonal antibody produced from hybridoma SM1A5 binds strongly to the N-mSMase ε denatured on the SDS-PAGE gel.

### EXAMPLE 6

### Physicochemical Characteristics of N-mSMase ε

### A. Kinetic Analysis

The N-mSMase ε prepared in Step 6 of Example 1 was subjected to Lineweaver-Burk analysis. In this regard, enzyme N-mSMase ε was used at amounts of 10 ng or less with the substrate sphingomyelin varying, in concentration, from 6.25 to 100 µM.

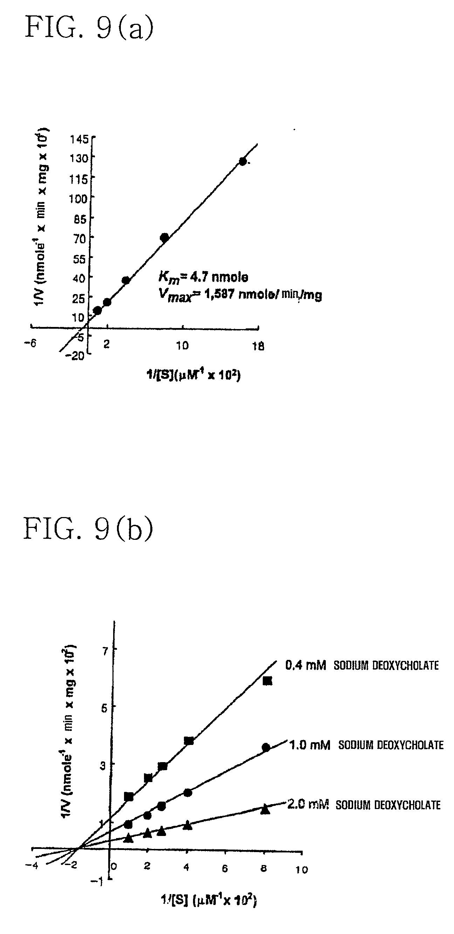

The results are depicted in Fig. 9a. As seen in the Lineweaver-Burk plot, the N-mSMase ε activity was traced in a typical straight line according to the Michaelis-Menten kinetics, showing a Km value of 47 µM and a Vmax greater than 1.587 nmol/min/mg.

Separately, the same Lineweaver-Burk analysis was carried out, but in the presence of 0.4, 1.0 and 2.0 mM sodium deoxycholate. The analysis results are given in Fig. 9b, which demonstrated that sodium deoxycholate could not change N-mSMase ε in the Km value for sphingomyelinase.

### B. Substrate Specificity

N-mSMase ε was examined for substrate specificity using [N-methyl-¹⁴C]sphingomyelin, 1,2-dipalmitoyl-3-phosphatidyl [N-methyl-³H] choline, phosphatidyl [2-³H] inositol 4,5-biphosphate, 1-stearoyl-2-[1-¹⁴C]arachidonoyl-sn-glycero-3-phosphocholine, and 1-acyl-2-[1-14C]arachidonoyl-sn-glycero-3-phosphoethanolamine.

With regard to these substrates, the N-mSMase activity was measured in the manner described in Experimental Example 1, being proven to have specificity for sphingomyelin 50-fold higher than for other substrates.

### C. Mg²⁺ Dependency

In order to examine the dependency of N-mSMase ε activity on Mg²⁺, Fraction B obtained in Step 6 of Example 1 was desalted by being applied to a PD-10 column (Sephadex G-25, Pharmacia Biotechnology Co., Sweden). For use, the column was equilibrated twice with distilled water, in advance. The desalted protein fraction (corresponding to protein 10 ng) was measured for N-mSMase ε activity in the presence of 1, 5, 10 and 25 mM of MgCl₂ in the manner described in Experimental Example 1.

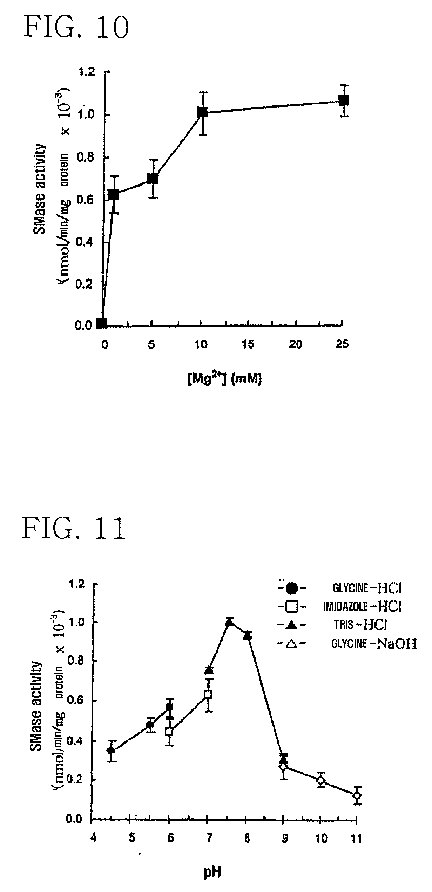

The results are given in Fig. 10. As seen in the plot, the N-mSMase ε activity is Mg²⁺-concentration dependent.

### D. Optimum pH

A reaction solution containing the desalted protein fraction (corresponding to protein 10 ng) obtained in C and 5 mM MgCl₂ was adjusted to pH 4.5, 5.5 or 6.0 with glycine-HCl, to pH 6.0 or 7.0 with imidazole-HCl, to 7.0, 7.5, 8.0 or 9.0 with Tris-HCl, or to pH 9, 10 or 11 with glycine-NaOH, followed by measuring enzyme activity in accordance with the procedure of Experimental Example 1.

The results are given in Fig. 11. As seen, the N-mSMase ε exhibited the highest activity in a neutral pH range with a sharp decrease in activity at pH lower than 6.0 or higher than 9.0. Accordingly, the N-mSMase ε of the present invention is a neutral-pH optimum enzyme.

### E. Influence of Other Cations

An examination was made of the influence of other cations, such as Ca²⁺, Fe³⁺, Fe²⁺, Zn²⁺, Cu²⁺ and Mg²⁺ on the activity of N-mSMase ε. To a solution containing the desalted protein fraction (corresponding to protein 10 ng) obtained in C and 5 mM MgCl₂, CaCl₂, FeCl₃, FeCl₂, ZnCl₂ or CuCl₂ was added to yield a final concentration of 0.01, 0.02, 0.05, 0.1, 0.25 or 0.5 mM, followed by the measurement of N-mSMase ε activity in accordance with the procedure of Experimental Example 1.

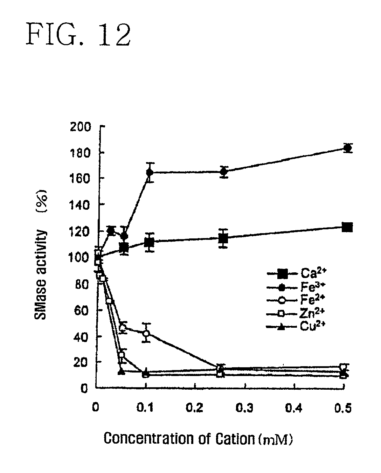

The results are given in Fig. 12. As seen in the plot, Ca²⁺ has no influence on the activity of N-mSMase ε at a concentration of 0.5 mM or less while Cu²⁺, Zn²⁺ and Fe²⁺ inhibit the enzyme activity with IC₅₀ values of 28 µM, 32 µM and 47 µM, respectively. On the other hand, Fe³⁺ has a positive influence, increasing the N-mSMase ε activity by a factor of 1.8.

### F. Inhibitors

The influence of reducing agents on the N-mSMase ε activity was examined.

The active fraction obtained in Step 6 of Example 1 (corresponding to 10 ng of protein) was added to 90 µl of Tris-HCl (pH 7.5) containing 0.5, 1, 2, 3, 4, 5, 7.5 or 10 mM of glutathione, DTT, or 2-mercaptoethanol and incubated at 37 °C for 10 min. After the addition of 10 µl of [N-methyl-¹⁴C]sphingomyelin, the N-mSMase activity was measured according to the procedure of Experimental Example 1. For comparison, a Tris-HCl buffer containing no reducing agents was used as a control. The N-mSMase ε activity influenced by the reducing agents was expressed as a percentage relative to the control.

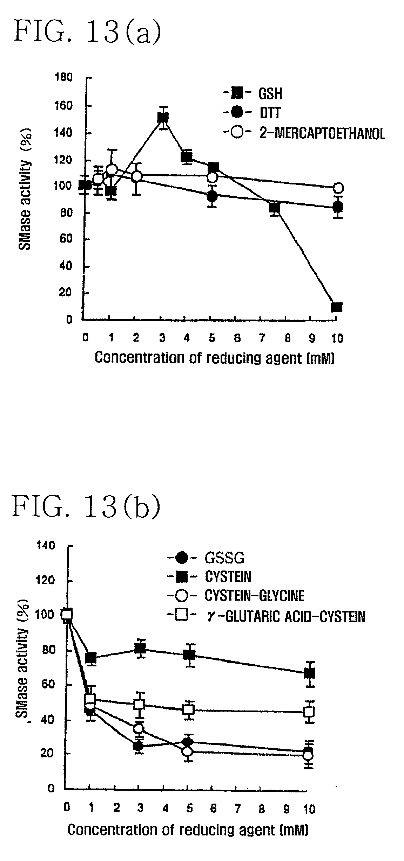

The results are given in Fig. 13a. Glutathione, as seen in Fig. 13a, has influence on the activity of N-mSMase ε. The activity shows a bimodal profile in which the activity of N-mSMase ε increases with increasing glutathione concentration to 3 mM, but decreases thereafter, finally being fully inhibited at a glutathione concentration of 10 mM. In contrast, the activity of N-mSMase ε was found to be not inhibited by DTT and 2-mercaptoethanol.

With the result of glutathione's influence, reducing agents similar to glutathione were also examined for their influence on the activity of N-mSMase ε. After being treated with 1, 3, 5 or 10 mM of GSSG, cystein, cystein-glycine or γ-glutaric acid-cystein, the activity of N-mSMase ε was measured in the same manner.

The results are given in Fig. 13b. The activity of N-mSMase ε was, as seen in Fig. 13b, found to be inhibited by GSSG, cystein, cystein-glycine or γ-glutaric acid-cystein.

### G. Effect of Surfactants

Sodium deoxycholate and Triton X-100 were selected as surfactants to be tested. The protein fraction obtained in C (corresponding to 10 ng of protein) was measured for N-mSMase ε activity in reaction solutions containing 0.5, 1, 2 and 5 mM of sodium deoxycholate, respectively, in accordance with the procedure of Experimental Example 1. In the case of Triton X-100, it was prepared at concentrations of 0.05, 0.01, 0.5 and 0.1 %.

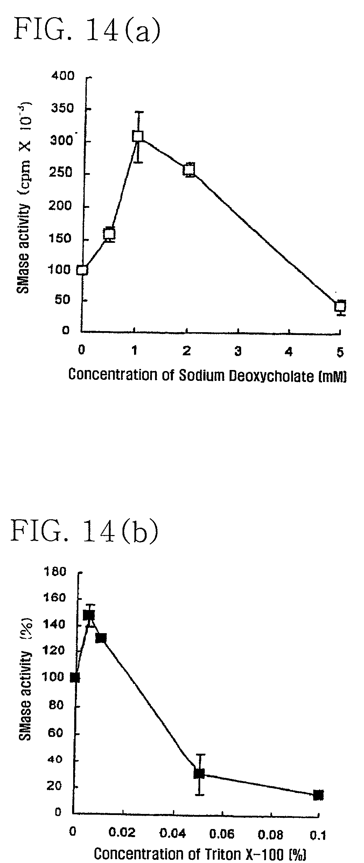

Effects of deoxycholate and Triton X-100 on the activity of the N-mSMase ε are depicted in Figs. 14a and 14b, respectively. As seen in these plots, the activity of the N-mSMase ε increases by 3 times in the presence of 1 mM sodium deoxycholate and by 1.5 times in the presence of 0.005 % Triton X-100.

### EXAMPLE 7

### Distribution of N-mSMase ε in Brain Tissue

From fetuses of Sprague-Dawley rats which were at 16 days gestation, cortex neuron cultures were prepared according to the teaching of Koh, J. Y., et al. (Exp, Neurol., 135, 153-159 (1996)) as follows. Cortex nervous tissues, extirpated from rat fetuses, were added in a Ca²⁺- and Mg²⁺-free Hank's balanced salt solution (HBSS) (1 mM sodium pyruvate and 10 mM HEPES, pH 7.4), after which free cells were isolated with the aid of a Pasture pipette which was narrowed by flame. They were plated on a DMEM (Dulbecco's Modified Eagle Medium)/F-12 (Gibco BRL, Grand Island, NY. U.S.A.) in a culture plate onto the bottom of which poly-D-lycine was coated, followed by incubation at 37 °C in a 5% CO₂ atmosphere. After 18 hours of incubation, 10 µM β-D-cytosine arabinofuranoside was added to the medium to prevent the growth of non-nerve cells.

Separately, from the brain neocortex of rats 1-3 days after the birth, a culture of neuroglial cells was prepared. This was plated on a 10% bovine fetal serum and 10% horse serum-supplemented medium which filled 0.25-0.5 of the well volume in a 6-well plate, followed by culturing for 2 hours. The medium was changed with a fresh one every week. The neuroglial cells were plated after 14-28 days of culturing.

After being washed three times with TBS, the cultured cortex neurons and neuroglial cells were each added in a homogenizing buffer (20 mM Tris-HCl pH 7.5, 1 mM EDTA, 10 mM 2-mercaptoethanol, 1 µg/ml leupeptin, and 1 mM phenylmethylsulfonyl fluoride) and sheared by 30 passages through a 26-gauge needle. Centrifuging the homogenate at 4°C at 2,000xg for 10 min afforded neuron debris and neuroglial cell debris. As for the neuroglial cell debris, it was centrifuged again at 4 °C at 100,000xg for 1 hour. While the supernatant was removed as a water-soluble fraction (S100), the pellet containing membranous materials (P100) was suspended in a homogenizing buffer with a half volume of the supernatant.

The neuron water-soluble fraction, the neuron membrane fraction, the neuroglial cell homogenate, and the N-mSMase ε obtained in Step 6 of Example 1 were electrophoresed together on 10% SDS-polyacrylamide gel. Using a 10,000-fold dilution of the antiserum obtained in Example 3 against the 60 kDa protein, Western blotting was carried out in the same manner as in Example 5.

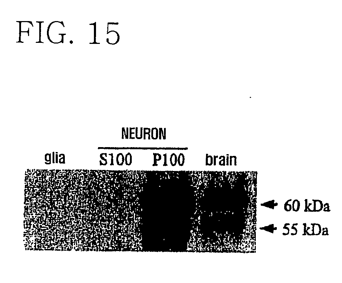

The results are given in Fig. 15. As apparent in the blot diagram, N-mSMase ε is situated in the neuron membrane fraction, but not in the neuron water-soluble fraction and the neuroglial cell homogenate.

Based on the results obtained above, the sphingomyelinase N-mSMase ε of the present invention is useful for the development of curing agents for brain diseases, such as Parkinson's disease, Alzheimer's diseases, etc., because of its location in the membrane of neurons alone. Additionally, the sphingomyelinase N-mSMase ε of the present invention finds useful applications in the search for its inhibitors and activating factors and the structural analysis of them by X-ray or NMR.

Meanwhile, the N-mSMase ε of the present invention can be examined for the functions and the intracellular modulation mechanism in which it is involved, by use of monoclonal antibodies specific therefor. These monoclonal antibodies are also useful for effectively isolating N-mSMase ε, as well as diagnosing cancers and degenerative diseases.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. A sphingomyelinase derived from the neuron membrane of the bovine brain, which is 60 kDa in molecular weight and Mg²⁺-dependent in activity with an optimal pH ranging from 6.0 to 9.0.

2. The sphingomyelinase as set forth in claim 1, wherein the activity of the sphingomyelinase is inhibited by glutathione with an IC₅₀ of 8.0 mM.

3. A method for isolating the sphingomyelinase of claim 1, comprising the steps of:
homogenizing a bovine brain tissue and centrifuging the homogenate to remove cell debris and nuclei;
separating cell membranes from the supernatant into a pellet through centrifugation;
treating the cell membrane pellet with a buffer containing ammonium sulfate to disassociate the sphingomyelinase from the membrane;
subjecting the extract to anion exchange chromatography;
sonicating the chromatography fraction in a buffer containing Triton X-100 and centrifuging the sonicated fraction to obtain a supernatant containing the sphingomyelinase; and
purifying the sphingomyelinase by subjecting the supernatant to hydrophobic interaction chromatography, anion exchange high-performance liquid chromatography, hydrophobic interaction high performance liquid chromatography, and cation exchange fast protein liquid chromatography, in due order.

4. The method as set forth in claim 3, wherein the anion exchange chromatography is carried out using a DEAE-cellulose column.

5. The method as set forth in claim 3, wherein the hydrophobic interaction chromatography is carried out using a butyl-Toyopearl 650 M column.

6. The method as set forth in claim 3, wherein the anion exchange high-performance liquid chromatography is carried out using a DEAE-5PW column.

7. The method as set forth in claim 3, wherein the hydrophobic high-performance liquid chromatography is carried out using a phenyl-5PW column.

8. The method as set forth in claim 3, wherein the cation exchange fast protein liquid chromatography is carried out using a mono-S fast protein liquid chromatography column.

9. A monoclonal antibody against the sphingomyelinase of claim 1, which is produced by Hybridoma SM1A5 (Deposition No. KCLRF-BT-00025).
